# EUROPÄISCHE PATENTSCHRIFT

(11) **EP 0 654 976 B1**
(45) Veröffentlichungstag und Bekanntmachung des Hinweises auf die Patenterteilung: **21.08.1996**
(21) Anmeldenummer: 94916127.7
(22) Anmeldetag: 09.06.1994
(51) Int. Cl.: A61B 17/00

(54) **VORRICHTUNG ZUM EINBRINGEN VON FIBRINKLEBER IN EINEN STICHKANAL**
DEVICE FOR THE INTRODUCTION OF FIBRIN SEALANT INTO A PUNCTURE CHANNEL
DISPOSITIF POUR L'INTRODUCTION DE COLLE A BASE DE FIBRINE DANS UN CANAL DE PONCTION

(30) Priorität: 16.06.1993 CH 1792/93
(43) Veröffentlichungstag der Anmeldung: 31.05.1995
(73) Patentinhaber: White Spot AG, CH-6342 Baar (CH)
(72) Erfinder: ILLI, Oskar, E., CH-8603 Schwerzenbach (CH)
(74) Vertreter: Feldmann, Clarence Paul
(86) Internationale Anmeldenummer: CH9400114
(87) Internationale Veröffentlichungsnummer: WO9428798

(56) Entgegenhaltungen:
- EP-A- 0 241 038
- EP-A- 0 443 256
- EP-A- 0 482 350
- FR-A- 2 378 528
- US-A- 1 882 213

## Beschreibung

Die vorliegende Erfindung betrifft eine Vorrichtung zur Einbringung von zweikomponenten Fibrinkleber in einen Stichkanal in die Nähe einer arteriellen oder venösen Punktionsstelle gemäß dem Oberbegriff des Patentanspruchs 1 wie es beispielsweise aus der EP-A-482350 bekannt ist.

Viele human- und veterinärmedizinische Eingriffe verlangen die Punktierung von Gefässen. Insbesondere bei perkutaner transluminarer Koronarangioplastie (PTCA), Herzoperationen und Herzkatheterisierungen müssen die punktierten Gefässe mit aller Vorsicht wieder verschlossen werden. In den meisten aller Fälle erfolgt dies mittels direkter Kompression von bis zu 1 Stunde und einem Druckverband, der bis zu 24 Stunden angelegt sein muss und eine 1 - 2 tägige Hospitalisation verlangt. Folglich besteht das Bestreben, eine Lösung zu finden, die zu einem schnelleren und sichereren Verschluss der Punktionsstelle führt.

Anlässlich eines Meetings der American Heart Association vom 17.11.1992 in New Orleans wurde eine Methode unter der Bezeichnung Vasoseal vorgestellt. Bei dieser Methode wurden jeweils zwei Kollagenpfropfen aus bovinem Kollagen in den Stichkanal bis an die Punktionsstelle eingestossen. Am erwähnten Meeting wurde festgehalten, dass neben der eher seltenen Abstossungsreaktion des körperfremden Kollagens diverse weitere Nachteile bzw. Risiken bestehen. So wurde festgehalten, dass dieses System in vielen Fällen ineffektiv ist und eine gewisse Emboliegefahr besteht. Bei rund 46% aller Fälle bildeten sich Hämatome in der Grössenordnung von 2 - 6 cm. Bis zur vollständigen Resorption des bovinen Kollagens vergehen Wochen bis Monate. Die Methode führt zudem zu einer vergrösserten Narbenbildung, die eine Ultraschallkontrolle erschwert. Schliesslich wurde die Hospitalisierung nicht überflüssig, aber zumindest um 24 Stunden verkürzt. Eines der wesentlichsten Probleme besteht jedoch in der Handhabung, d.h. die Einführung der Kollagenpfropfen in den Stichkanal. Da nacheinander zwei Kollagenpfropfen in den Stichkanal eingedrückt werden müssen, ist beispielsweise die Eindringtiefe für den Anwender schwer feststellbar. Werden die Kollagenpfropfen zu tief eingestossen, so kann der Kollagenpfropfen durch die Punktionsstelle in das Gefäss eingeschoben werden, was zu einem Verschluss führen würde oder das Gefäss selber zudrücken.

Es hat sich gezeigt, dass bei der Verwendung von aus Bluteiweiss gewonnenem, körpereigenem Blutgerinnungsmittel in der Form von Zweikomponenten-Fibrinkleber, deren Komponenten man im Moment der Zuführung vermischt und diese Mischung während oder direkt im Anschluss an einen intravasalen Eingriff in den Stichkanal möglichst gefässnah einbringt, ein optimaler Gefässverschluss entsteht. Histologische Untersuchungen haben diesen Sachverhalt bestätigt.

Es war folglich die Aufgabe der vorliegenden Erfindung, eine Vorrichtung zu schaffen, mittels der Fibrinkleber in einen Stichkanal in die Nähe einer arteriellen oder venösen Punktionsstelle gebracht werden kann.

Die Lösung der Aufgabe erfolgt durch die Merkmale des Patentanspruchs 1.

Weitere vorteilhafte Ausgestaltungsformen der Vorrichtung gehen aus den abhängigen Ansprüchen hervor und deren Bedeutung und Vorteile sind in der nachfolgenden Beschreibung dargelegt.

In den beiliegenden Zeichnungen sind zwei bevorzugte Ausführungsbeispiele der erfindungsgemässen Vorrichtung dargestellt und anhand der Beschreibung erläutert. Es zeigt:
- Figur 1: eine Gesamtansicht einer ersten Ausführungsform der erfindungsgemässen Vorrichtung in der Gesamtansicht und
- Figur 2: einen Querschnitt durch die Vorrichtung nach Figur 1 entlang der Linie II-II in grösserem Massstab.
- Figur 3: einen achsialen Längsschnitt durch die Ausführung gemäss Figur 1 und
- Figur 4: einen vergrösserten Ausschnitt der Vorrichtung im Bereich des Anschlussstutzens und
- Figur 5: einen vergrösserten Ausschnitt im Bereich des Austritts der Arbeitskanüle aus der Versiegelungskanüle.
- Figur 6: zeigt wiederum eine Gesamtansicht einer zweiten Ausführungsform der Vorrichtung und
- Figur 7: einen Querschnitt durch diese Vorrichtung entlang der Linie VII-VII gemäss Figur 6.
- Figur 8: stellt einen achsialen Längsschnitt durch die Vorrichtung nach Figur 6 dar und
- Figur 9: wiederum einen vergrösserten Ausschnitt aus der Zeichnung nach Figur 8 im Bereich des Anschlussstutzens und
- Figur 10: im Bereich des Austrittes der Arbeitskanüle aus der Versiegelungskanüle.

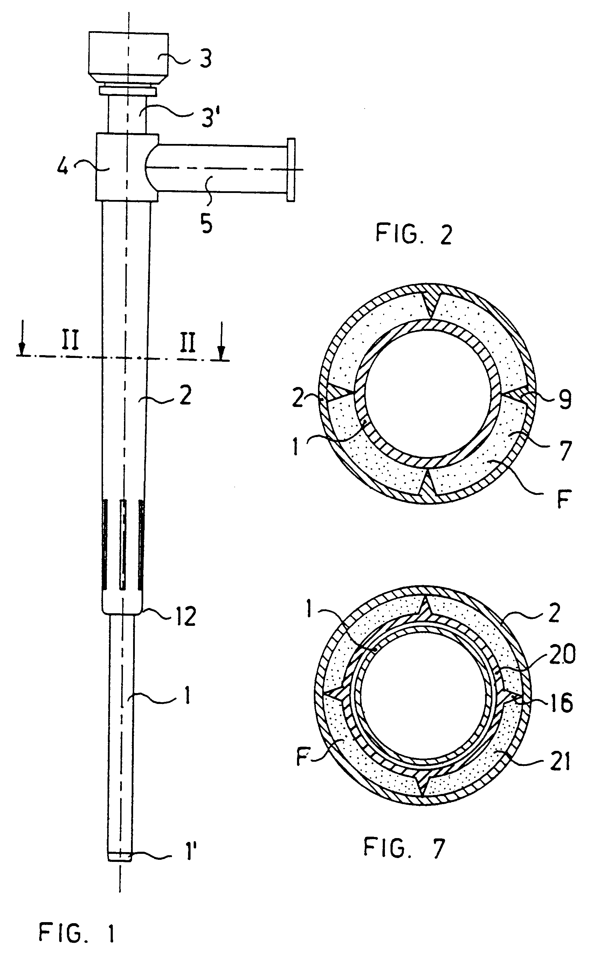

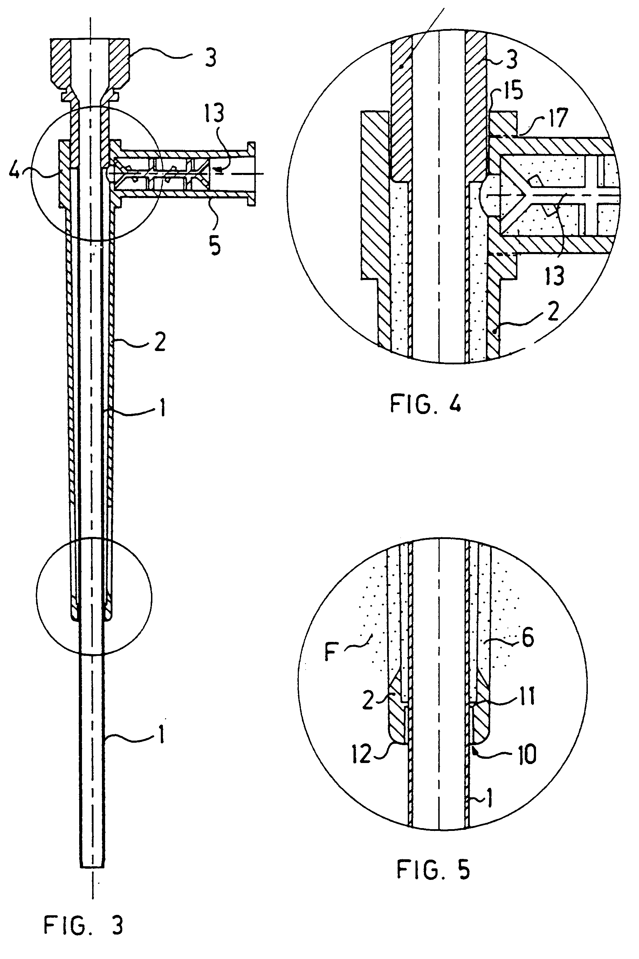

Nachfolgend werden vorerst die beiden bevorzugten Ausführungsformen der erfindungsgemässen Vorrichtung und danach deren Handhabung und die generelle Verwendung von Fibrinkleber zur Versiegelung einer Punktionsstelle eines Gefässes dargestellt. Die einfachere Ausführungsform der Vorrichtung gemäss den Figuren 1 bis 5 besteht lediglich aus drei zusammenfügbaren Teilen. Mit dem Bezugszeichen 1 ist die Arbeitskanüle, in der Fachsprache auch Worksheet genannt, bezeichnet. Die Arbeitskanüle selber ist lediglich ein an beiden Enden offenes, zylindrisches Röhrchen aus Kunststoff. Dessen vorderes Ende 1' dient der Einführung der Kanüle durch die Punktionsstelle in das geöffnete Blutgefäss. Die Arbeitskanüle ist relativ dünnwandig und weist demzufolge eine gewisse Biegeelastizität auf. In dieser Ausgestaltungsform ist die Arbeitskanüle 1 am anderen Ende, dem rückwärtigen Ende, fest mit einer Medizinalkupplung 3 verbunden. Die eigentliche Medizinalkupplung 3 kann beispielsweise eine bekannte Luerlock-Kupplung sein. An der eigentlichen Medizinalkupplung 3 ist eine exakt gearbeitete, formschlüssig und dichtend passende Muffe 3' angeformt. Die Arbeitskanüle 1 verläuft in Längsrichtung achsial durch eine Versiegelungskanüle 2 und ragt unten ein Stück weit aus der Versiegelungskanüle heraus. Die Versiegelungskanüle 2 ist selber auch wieder in der Form eines konzentrischen Röhrchens gestaltet, doch ist dessen Innendurchmesser, vorzugsweise jedoch auch deren Aussendurchmesser, von oben nach unten abnehmend, d.h. der Innendurchmesser nimmt von der Seite, an der die Medizinalkupplung 3 eingeschoben wird bis zum unteren Ende, wo die Arbeitskanüle aus der Versiegelungskanüle 2 heraustritt, ab.

Über die gesamte Länge, auf der die Arbeitskanüle 1 von der Versiegelungskanüle 2 konzentrisch umgeben ist, verbleibt somit zwischen der Arbeitskanüle und der Versiegelungskanüle ein Hohlraum 7. Am oberen Ende weist die Versiegelungskanüle 2 eine verstärkte Manschette 4 auf, die eine erheblich dickere Wandstärke als die Wandstärke der Versiegelungskanüle 2 hat. Im Bereich der verstärkten Manschette 4 mündet ein Ansatzstutzen 5 in die Versiegelungskanüle 2. Durch diesen Ansatzstutzen 5 lässt sich ein Zweikomponenten-Fibrinkleber in den Hohlraum 7 zwischen Arbeitskanüle und Versiegelungskanüle einführen. Der Fibrinkleber kann aus dem Hohlraum 7 nur durch die Austrittsöffnungen 6 am unteren Bereich der Versiegelungskanüle 2 austreten. Damit nicht unbeabsichtigterweise Fibrinkleber in das Blutgefäss eindringt, sind die Austrittsöffnungen 6 mindestens annähernd radial nach aussen gerichtet. Selbstverständlich ist unter radial nicht nur die im streng geometrischen Sinne verstandene Richtung gemeint. Vielmehr soll hiermit lediglich zum Ausdruck gebracht werden, dass die Ausströmungsrichtung nicht axsial ist. Selbstverständlich wird die Funktion der Vorrichtung durch mehrere am Umfang verteilte Austrittsöffnungen 6 sichergestellt. Aus fertigungstechnischen Gründen wird man diese in der Gestalt von mehreren am Umfang verteilten Längsschlitzen formen.

Die obere Öffnung der Versiegelungskanüle 2 muss als exakte Aufnahme 15 für die Medizinalkupplung 3 mit Passitz versehen sein.

Um die Arbeitskanüle 1 im Bereich der Durchtrittsöffnung 10 gegenüber der Versiegelungskanüle 2 abzudichten, ist in der Durchtrittsöffnung 10 eine radial nach innen gerichtete, dichtend an der Aussenfläche der Arbeitskanüle 1 anliegende, ringförmige Dichtwulst oder Dichtrippe 11 angebracht.

Obwohl bevorzugterweise der Ansatzstutzen 5 einstückig direkt im Bereich der verstärkten Manschette an der Versiegelungskanüle 2 angeformt ist, ist es selbstverständlich auch möglich, den Ansatzstutzen getrennt zu fertigen und nachträglich beispielsweise dank einem Gewinde 17 mit der Versiegelungskanüle zu verbinden. Statt der Schraubverbindung 17 ist selbstverständlich auch eine Schweiss- oder Klebverbindung denkbar. Für die Vermischung der beiden Komponenten des Zweikomponenten-Fibrinklebers sind auf dem Markt bereits Mischorgane erhältlich. Aus Kostengründen wird man folglich den Ansatzstutzen 5 so dimensionieren, dass in denselben ein bereits erhältliches Mischorgan 13 einschiebbar ist.

Wie bereits erwähnt ist die Wanddicke der Arbeitskanüle 1 sehr gering. Sie beträgt vorzugsweise lediglich einige Zehntelmillimeter. Auch der konzentrisch um die Arbeitskanüle 1 zwischen deren Aussenwand und der Innenwand der Versiegelungskanüle 2 verbleibende Hohlraum 7 ist äusserst klein dimensioniert. Da durch die Arbeitskanüle 1 das gesamte chirurgische Instrumentarium ein und ausgestossen werden muss, ist es von Vorteil, Mittel zu sehen, die bewirken, dass dieser Hohlraum 7 durchgehend offen bleibt. Hierzu sind an der Innenwand der Versiegelungskanüle 2 Stützrippen 9, die vorzugsweise achsial verlaufen, angeordnet.

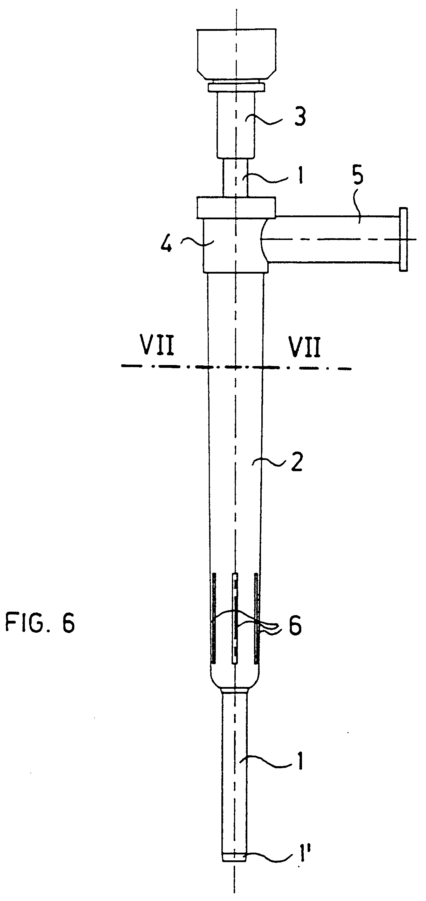

Die Stützrippen 9 ergeben auch eine Versteifung der ebenfalls dünnwandigen Versiegelungskanüle 2. Hiermit wird auch die Gefahr, dass eine geringfügige Kontraktion des Muskelgewebes, das die Versiegelungskanüle hindurchverläuft, zu einer Deformation der Versiegelungskanüle 2 führt, welche den Hohlraum 7 verschliessen könnte, ausgeschlossen. Auf diese Weise ist der erforderliche Durchgang für den Fibrinkleber auf jeden Fall gesichert. Die zweite bevorzugte Ausführungsform der erfindungsgemässen Vorrichtung zur Einbringung von Zweikomponenten-Fibrinkleber in einen Stichkanal in die Nähe einer arteriellen oder venösen Punktionsstelle ist in den Figuren 6 bis 10 dargestellt. Während bei der ersten Ausführungsform die Arbeitskanüle 1 mit der dazugehörigen Medizinalkupplung 3 exakt auf die Versiegelungskanüle zur dichtenden Verbindung angepasst sein muss, kann bei der zweiten Ausführungsform eine handelsübliche Arbeitskanüle mit einer beliebigen Medizinalkupplung 3 verwendet werden. In diesem Fall entfällt eine dichtende Verbindung zwischen der Medizinalkupplung 3 und der Versiegelungskanüle 2.

In der äusseren Gestaltungsform ist zwischen den beiden Vorrichtungen kaum ein Unterschied ersichtlich. Entsprechend sind bei beiden Ausführungsformen identische Teile mit gleichen Bezugszahlen versehen. Auch hier wird wiederum die Versiegelungskanüle 2 von der Arbeitskanüle 1 in achsialer Richtung vollständig durchsetzt. Die an der Arbeitskanüle 1 fest angeordnete Medizinalkupplung 3 greift jedoch nicht in die Versiegelungskanüle 2 ein, sondern liegt kurz oberhalb dieser. Das untere Ende der Arbeitskanüle 1' ist wiederum konisch zulaufend gestaltet.

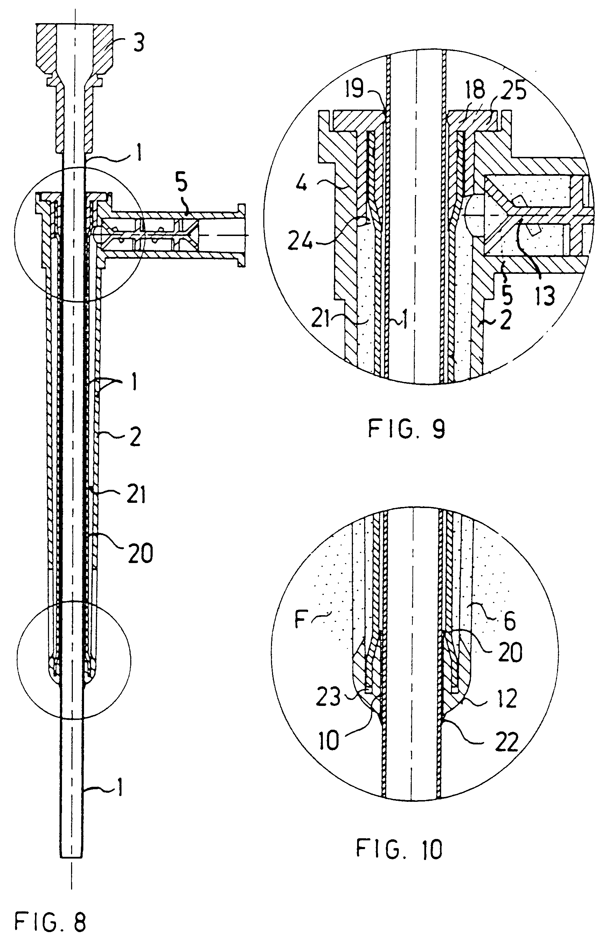

Die Versiegelungskanüle 2 hat wiederum eine verstärkte Manschette 4 an ihrem oberen Ende. Im Bereich der verstärkten Manschette 4 mündet auch hier wiederum ein Ansatzstutzen 5 in den Innenraum der Versiegelungskanüle 2. Diese hat wiederum am unteren Ende, d.h. im Bereich kurz oberhalb der Durchtrittsöffnung 10, mindestens eine etwa radial nach aussen gerichtete Austrittsöffnung 6. Auch hier sind mehrere gleichmässig am Umfang verteilte Austrittsöffnungen 6 angebracht, die wiederum schlitzförmig gestaltet sind. Der wesentliche Unterschied dieser Ausführung gegenüber der vorbeschriebenen Ausgestaltungsform geht insbesondere aus der Schnittzeichnung gemäss der Figur 8 hervor. In der Versiegelungskanüle 2 ist diesmal eine Stützhülle 20 gehalten, die einen Freiraum 21 zwischen sich und der Innenwand der Versiegelungskanüle 2 definiert. Die, die Versiegelungskanüle 2 durchsetzende Arbeitskanüle 1 verläuft nun innerhalb der Stützhülle 20. Der durch den Ansatzstutzen 5 eingepresste Fibrinkleber verläuft nun nicht mehr direkt zwischen der Aussenwand der Arbeitskanüle 1 und der Innenwand der Versiegelungskanüle 2, sondern zwischen der Aussenwand der Stützhülle 20 und der Innenwand der Versiegelungskanüle 2. Die Versiegelungshülle 20 ist im Bereich der unteren Durchtrittsöffnung 10 mit einem verdickten Kopfbereich 12 versehen. Dies trifft auch bei der erst beschriebenen Ausführungsform zu. In diesen verdickten Kopfbereich 12 ist innen eine konzentrische Nut 23 eingelassen. Diese Nut 23 verengt sich von oben nach unten, so dass die Stützhülle 20 beim Einschieben leicht aufgeweitet wird und dichtend in der Ringnut 23 zu liegen kommt. Auf ähnliche Weise ist die Stützhülle 20 oben in einen Durchführungsstopfen 18 gehalten. Der Durchführungsstopfen 18 weist eine zentrische Durchgangsbohrung 24 auf.

Durch diese Bohrung tritt die Arbeitskanüle 1 in die Stützhülle 20 ein. Eine ringförmige Dichtwulst 25 führt zu einer klemmenden und dichtenden Halterung der Arbeitskanüle 1 in der Stützhülle 20. Der Durchführungsstopfen 18 ist mit einem Kragen 25 versehene der im montierten Zustand des Durchführungsstopfens vollständig in eine Ausnehmung der verstärkten Manschette 4 hineinpasst. Um eine genügende Wandstärke zu erhalten, kann die verstärkte Manschette 4 im oberen Bereich mit einem nochmals verstärkteren Aussendurchmesser versehen sein. Auch der Durchführungsstopfen 18 ist mit einer ringförmigen, konzentrischen Nut 24 versehen, die von unten nach oben sich im Durchmesser erweitert, so dass auch hier die Stützhülle 20 leicht aufgeweitet klemmend und dichtend gehalten wird. Wie aus der Figur 9 deutlich ersichtlich ist, tritt der Zweikomponenten-Fibrinkleber durch den Anschlussstutzen 5, in dem das Mischorgan 13 angeordnet ist, in den Freiraum 21 ein.

Auch hier ist der Kopfbereich 12 sprunghaft verdickt und gerundet. Die sprunghafte Verdickung dient dazu, dass die Versiegelungskanüle 2 nicht durch die Punktierungsstelle im Blutgefäss in dieses eingestossen wird. Die Rundung andererseits soll die Einführung der Versiegelungskanüle in den Stichkanal erleichtern.

Auch hier ist die Arbeitskanüle 1 zur Versiegelungskanüle 2 abgedichtet. Dies erfolgt hier mittels einer endständigen Dichtlippe 22, die auf der Aussenwand der Arbeitskanüle 1 aufliegt.

Die Funktion der ringförmigen Dichtlippe 22 ist jedoch nicht dieselbe wie diejenige des Dichtwulst 11 bzw. des Dichtringes nämlich zur Abdichtung des Hohlraumes der Versiegelungskanüle 2 und damit zur Verhinderung des Austrittes von Fibrinkleber in achsialer Richtung, sondern dient der Verhinderung des Eintrittes von Blut im Bereich zwischen der Arbeitskanüle 1 und der Stützhülle 20.

Nachfolgend sei noch die Benutzung der erfindungsgemässen Vorrichtung kurz beschrieben. Bei der Katheterisierung wird in einem ersten Arbeitsgang eine Hohlnadel durch die Haut und den verschiedenen darunterliegenden Gewebeschichten bis in das zu punktierende Blutgefäss eingestossen. Durch die Hohlnadel wird ein Mandrin in das Blutgefäss vorgeschoben. Unter Belassung des Mandrins in der eingeschobenen Lage wird die Hohlnadel über das Mandrin zurückgezogen und dafür über dasselbe ein Dilatator in den Stichkanal bis in das Blutgefäss vorgeschoben. Über den Dilatator wird danach die Arbeitskanüle und die Versiegelungskanüle geschoben, wobei die Arbeitskanüle bis in das Blutgefäss eingeschoben wird, während die Versiegelungskanüle mit ihrem sprunghaft verdickten Kopfbereich lediglich bis zur Punktionsstelle vorgeschoben wird. Die Austrittsöffnungen 6 liegen somit oberhalb der Punktionsstelle des Blutgefässes, aber innerhalb des Stichkanales. Der Arzt kann nun durch die Arbeitskanüle das erforderliche Instrumentarium in das Blutgefäss einschieben. Dies kann ein Ballonkatheter sein, ein Glasfaserlichtleiter oder die Sonde einer Kamera oder nochmals andere Mittel.

Nach Abschluss des Eingriffes oder der Untersuchung wird zuerst das Instrumentarium durch die Arbeitskanüle aus dem Gefäss herausgezogen, und darauf durch den Anschlussstutzen 5 dem Freiraum 21 oder dem Hohlraum 7 der zweikomponenten Fibrinkleber durch die Austrittsöffnungen 6 in den Stichkanal gepresst. Schon nach wenigen Sekunden führt der Fibrinkleber zu einer Gerinnung des im Bereich des Stichkanales bzw. der Punktionsstelle zu einem Fibrinclot, wodurch die Blutung sogleich unterbunden ist. Die Bildung von Hämatomen wird vollständig unterbunden. Ein Embolierisiko konnte nicht mehr festgestellt werden. In allen bisher durchgeführten Versuchen wurde eine hundertprozentige Effektivität erreicht.

Abstossreaktionen des humanen Fibrinklebers wurden nicht festgestellt. Auch bei der Verwendung einer erhöhten Konzentration von Aprotinin wurde eine einwandfreie Versiegelung im Tierversuch (Hund, Minipig) erreicht.

Eine Hospitalisierung beim Patienten kann somit entfallen.

Der vorliegende Applikator erlaubt eine gefahrlose Verwendung des Fibrinklebers.

Neben den vorher beschriebenen vorzugsweisen Ausführungsformen der erfindungsgemässen Vorrichtung sind sicherlich noch weitere Ausführungsformen denkbar, ohne dabei den grundlegenden Erfindungsgedanken zu verlassen.

## Patentansprüche

1. Vorrichtung zur Einbringung von Zweikomponenten-Fibrinkleber in einen Stichkanal in die Nähe einer arteriellen oder venösen Punktionsstelle, wobei diese eine Versiegelungskanüle (2) umfasst, die von einer Arbeitskanüle von oben nach unten axial durchsetzt ist, die zur intravasalen Einführung eines Instrumentariums in ein Gefäss dient, dadurch gekennzeichnet, dass die Arbeitskanüle (1) von der Versiegelungskanüle (2) distanziert umgeben ist, so dass bei Verwendung der Vorrichtung der Fibrinkleber von einem Anschlussstutzen (5) zu mehreren, schlitzförmigen radial gerichteten Austrittsöffnungen (6) in der Versiegelungskanüle zwischen dieser und der Arbeitskanüle geführt ist.

2. Vorrichtung nach Anspruch 1, dadurch gekennzeichnet, dass die Versiegelungskanüle (2) am oberen Ende mit einer einstückig angeformten verstärkten Manschette (4) versehen ist.

3. Vorrichtung nach Anspruch 2, dadurch gekennzeichnet, dass der Anschlussstutzen (5) an der verstärkten Manschette (4) seitlich einstückig angeformt ist.

4. Vorrichtung nach Anspruch 2, dadurch gekennzeichnet, dass der Anschlussstutzen (5) gesondert gefertigt und mit der gestärkten Manschette verbindbar (17) ist.

5. Vorrichtung nach den Ansprüchen 3 oder 4, dadurch gekennzeichnet, dass im Anschlussstutzen (5) ein Mischorgan (13) einschiebbar gehalten ist.

6. Vorrichtung nach Anspruch 1, dadurch gekennzeichnet, dass sich der innere Durchmesser der Versiegelungskanüle (2) vom Bereich der Einmündung des Anschlussstutzens zu den Ausgangsöffnungen (6) hin verringert.

7. Vorrichtung nach Anspruch 1, dadurch gekennzeichnet, dass die Versiegelungskanüle (2) nach innen gerichtete Stützrippen (9) aufweist, die einen Hohlraum (7) zur Führung des Fibrinklebers (F) vom Anschlussstutzen (5) zu den Austrittsöffnungen (6) gewährleisten.

8. Vorrichtung nach Anspruch 7, dadurch gekennzeichnet, dass die Stützrippen (9) mindestens annähernd radial verlaufend ausgerichtet sind.

9. Vorrichtung nach Anspruch 2, dadurch gekennzeichnet, dass die Arbeitskanüle (7) fest mit einer Medizinalkupplung (3) verbunden ist, die kraft- und/oder formschlüssig dichtend, axial zur Arbeitskanüle (1) verlaufend, in der verstärkten Manschette (4) der Versiegelungskanüle (2) gehalten (15) ist.

10. Vorrichtung nach Anspruch 9, dadurch gekennzeichnet, dass die Versiegelungskanüle (2) im Bereich des unteren Durchtritts (10) der Arbeitskanüle (1) durch die Versiegelungskanüle (2) diese einen radial nach innen gerichteten ringförmigen Dichtwulst (11) aufweist.

11. Vorrichtung nach Anspruch 2, dadurch gekennzeichnet, dass die Versiegelungskanüle (2) oben mit einem Durchführungsstopfen (18) verschlossen ist, in dem eine rohrförmige Stützhülle (20) dichtend gehalten ist, die selber wiederum die gesamte Versiegelungskanüle (2) durchsetzt und am gegenüberliegenden, unteren Ende in der Versiegelungskanüle (2) dichtend gehalten ist, und dass die Arbeitskanüle (1) in der Stützhülle (20) frei beweglich geführt ist, so dass der Fibrinkleber (F) im radialen Freiraum (21) zwischen Stützhülle (20) und Versiegelungskanüle (2) vom Anschlussstutzen (5) bis zu den Austrittsöffnungen (6) fliessen kann.

12. Vorrichtung nach Anspruch 11, dadurch gekennzeichnet, dass die Stützhülle (20) mit mehreren, radial nach aussen gerichteten, zur Innenwand der Versiegelungskanüle (2) gerichteten Versteifungsrippen (16) versehen ist.

13. Vorrichtung nach Anspruch 1, dadurch gekennzeichnet, dass die Versiegelungskanüle (2) am unteren Ende, an dem die Arbeitskanüle (1) aus der Versiegelungskanüle (2) austritt, einen sprunghaft erweiterten, gerundeten Kopfbereich (12) aufweist.

14. Vorrichtung nach Anspruch 12, dadurch gekennzeichnet, dass der Kopfbereich (12) eine axsial verlaufende, ringförmige, auf der Arbeitskanüle aufliegende Dichtungslippe (22) aufweist.

## Claims

1. Device for introducing two-component fibrin sealant into a puncture channel in the vicinity of an arterial or venous puncture point, the latter comprising a sealing cannula (2), which is axially traversed from top to bottom by a working cannula, which is used for the intravascular introduction of instruments into a vessel, characterized in that the working cannula (1) is surrounded in spaced manner by the sealing cannula (2), so that when using the device the fibrin sealant is guided from a connecting stub (5) to several slot-like, radially directed outlets (6) in the sealing cannula between the latter and the working cannula.

2. Device according to claim 1, characterized in that at the upper end the sealing cannula (2) is provided with a one-piece shaped, reinforced sleeve (4).

3. Device according to claim 2, characterized in that the connecting stub (5) is laterally shaped in one piece onto the reinforced sleeve (4).

4. Device according to claim 2, characterized in that the connecting stub (5) is separately manufactured and is connectable (17) to the reinforced sleeve.

5. Device according to claim 3 or 4, characterized in that a mixing member (13) is insertably held in the connecting stub (5).

6. Device according to claim 1, characterized in that the internal diameter of the sealing cannula (2) decreases from the vicinity of the mouth of the connecting stub to the outlets (6).

7. Device according to claim 1, characterized in that the sealing cannula (2) has inwardly directed support ribs (9), which ensure a cavity (7) for guiding the fibrin sealant (F) from the connecting stub (5) to the outlets (6).

8. Device according to claim 7, characterized in that the support ribs (9) are oriented at least approximately radially.

9. Device according to claim 2, characterized in that the working cannula (7) is fixed to a medical connector (3), which running in positively and/or non-positively sealing manner and axially with respect to the working cannula (1) is held (15) in the reinforced sleeve (4) of the sealing cannula (2).

10. Device according to claim 9, characterized in that the sealing cannula (2) has in the vicinity of the lower passage (10) of the working cannula (1) through the sealing cannula (2) a radially inwardly directed, circular sealing bead (11).

11. Device according to claim 2, characterized in that the sealing cannula (2) is closed at the top by a passage plug (18), in which is sealingly held a tubular support cover (20), which itself traverses the entire sealing cannula (2) and is sealingly held in the sealing cannula (2) at the opposite, lower end, and that the working cannula (1) is guided in freely movable manner in the support cover (20), so that the fibrin sealant (F) can flow in the radial free space (21) between the support cover (20) and the sealing cannula (2) from the connecting stub (5) to the outlets (6).

12. Device according to claim 11, characterized in that the support cover (20) is provided with several, radially outwardly directed reinforcing ribs (16) directed towards the inner wall of the sealing cannula (2).

13. Device according to claim 1, characterized in that at the lower end at which the working cannula (1) passes out of the sealing cannula (12), the latter has a suddenly widened, rounded head region (12).

14. Device according to claim 12, characterized in that the head region (12) has an axially directed, circular sealing lip (22) bearing on the working cannula.

## Revendications

1. Dispositif pour introduire de la colle à deux composants à base de fibrine dans un canal de ponction au voisinage de l'endroit d'une ponction artérielle ou veineuse, ce dispositif comprenant une canule d'obturation (2) qui est traversée axialement de haut en bas par une canule de travail, laquelle sert à introduire un instrument dans un vaisseau sanguin par voie intravasculaire, caractérisé par le fait que la canule de travail (1) est entourée à distance par la canule d'obturation (2), de sorte que, lors de l'utilisation du dispositif, la colle à base de fibrine est amenée dans la canule d'obturation, entre celle-ci et la canule de travail, depuis une tubulure de raccordement (5) jusqu'à plusieurs ouvertures de sortie (6) en forme de fentes dirigées radialement.

2. Dispositif selon la revendication 1, caractérisé par le fait que la canule d'obturation (2) est pourvue à son extrémité supérieure d'un manchon renforcé (4) qui est formé d'un seul tenant.

3. Dispositif selon la revendication 2, caractérisé par le fait que la tubulure de raccordement (5) est formée latéralement d'un seul tenant sur le manchon renforcé (4).

4. Dispositif selon la revendication 2, caractérisé par le fait que la tubulure de raccordement (5) est fabriquée séparément, et qu'elle peut être reliée (17) au manchon renforcé.

5. Dispositif selon la revendication 3 ou 4, caractérisé par le fait qu'un organe mélangeur (13) est maintenu dans la tubulure de raccordement (5) en pouvant y être enfoncé.

6. Dispositif selon la revendication 1, caractérisé par le fait que le diamètre intérieur de la canule d'obturation (2) diminue depuis la zone de l'embouchure de la tubulure de raccordement jusqu'aux ouvertures de sortie (6).

7. Dispositif selon la revendication 1, caractérisé par le fait que la canule d'obturation (2) présente des nervures d'appui (9) qui sont dirigées vers l'intérieur et qui assurent la présence d'un espace creux (7) destiné à amener la colle à base de fibrine (F) de la tubulure de raccordement (5) aux ouvertures de sortie (6).

8. Dispositif selon la revendication 7, caractérisé par le fait que les nervures d'appui (9) sont dirigées de manière à s'étendre radialement, du moins approximativement.

9. Dispositif selon la revendication 2, caractérisé par le fait que la canule de travail (1) est solidaire d'un raccord à usage médical (3) qui est maintenu (15) d'une manière étanche dans le manchon renforcé (4) de la canule d'obturation (2), par conjugaison des forces et/ou des formes, en s'étendant axialement par rapport à la canule de travail (1).

10. Dispositif selon la revendication 9, caractérisé par le fait que la canule d'obturation (2) présente, dans la région inférieure (10) de passage de la canule de travail (1) à travers la canule d'obturation (2), un bourrelet d'étanchéité annulaire (11) qui est dirigé radialement vers l'intérieur.

11. Dispositif selon la revendication 2, caractérisé par le fait que la canule d'obturation (2) est fermée à sa partie supérieure par un bouchon de passage (18) dans lequel est maintenue d'une manière étanche une gaine d'appui tubulaire (20) qui traverse elle-même à son tour la totalité de la canule d'obturation (2) et qui est maintenue d'une manière étanche dans la canule d'obturation (2) à l'extrémité inférieure opposée, et par le fait que la canule de travail (1) est guidée dans la gaine d'appui (20) en étant librement mobile, de sorte que la colle à base de fibrine (F) peut s'écouler depuis la tubulure de raccordement (5) jusqu'aux ouvertures de sortie (6) dans l'espace libre radial (21) qui est compris entre la gaine d'appui (20) et la canule d'obturation (2).

12. Dispositif selon la revendication 11, caractérisé par le fait que la gaine d'appui (20) est pourvue de plusieurs nervures de raidissement (16) qui sont dirigées radialement vers l'extérieur et qui s'étendent vers la paroi intérieure de la canule d'obturation (2).

13. Dispositif selon la revendication 1, caractérisé par le fait que la canule d'obturation (2) présente, à son extrémité inférieure où la canule de travail (1) sort de la canule d'obturation (2), une zone de tête arrondie (12) qui s'élargit brusquement.

14. Dispositif selon la revendication 13, caractérisé par le fait que la zone de tête (12) présente une lèvre d'étanchéité annulaire (22) qui s'étend axialement et qui porte sur la canule de travail.
